# EUROPEAN PATENT SPECIFICATION

(11) **EP 3 157 359 B1**
(45) Date of publication and mention of the grant of the patent: **01.01.2020**
(21) Application number: 15803868.7
(22) Date of filing: 01.06.2015
(51) Int. Cl.: A23L 2/56, A23G 1/30, A23G 3/36, A23G 9/32, A23P 10/30, A23L 3/30, A23L 35/00, A23L 27/00, A23L 33/00, A23L 2/00

(54) **AFFECTING ENJOYMENT OF FOOD**
GENUSSBEEINFLUSSUNG VON LEBENSMITTELN
INFLUENCE SUR LE PLAISIR ALIMENTAIRE

(30) Priority: 05.06.2014 US 201414297196
(43) Date of publication of application: 26.04.2017
(73) Proprietor: Jb Scientific, LLC, New York, NY 10028-0208 (US)
(72) Inventor: MANNE, Joseph, New York, New York 10028 (US)
(74) Representative: Carvajal y Urquijo, Isabel
(86) International application number: PCT/US2015/033558
(87) International publication number: WO 2015/187568

(56) References cited:
- WO-A1-2007/017742
- WO-A2-2005/099663
- WO-A2-2013/144836
- US-A- 5 635 229
- US-A- 6 053 738
- US-A1- 2002 006 455
- US-A1- 2003 129 757
- US-A1- 2005 167 521
- US-A1- 2009 216 070
- US-A1- 2013 270 267
- US-A1- 2013 302 477
- US-B2- 6 803 987
- US-B2- 7 376 344
- US-B2- 7 562 816
- US-B2- 7 972 646

## Description

### Field of the Invention

This invention relates to food, and more particularly to affecting enjoyment of food by providing an increased enjoyment of food, or a decreased enjoyment of food.

### Related Art

Enhanced enjoyment of food by the addition of flavors or aroma to food or to the packaging for the food is known.

One such category is where a food is provided with a separate scent or aroma where the consumer adds the scent or aroma directly into the food itself. In this category there is a separate aroma or scent package that is opened and then a scent containing compound is added to the food prior to or during the process of ingestion. There is one scent containing compound and it is added directly into the food or beverage being consumed or it is added to the container from which the food is being ingested. This allows for the scent or aroma to be appreciated during consumption. The consumer can choose the scent they want to use.

Another category is a food where the scent or aroma is incorporated into the packaging or utensil for the food so that it provides scent when the package or utensil is near the mouth and nose.

A third category is a food which is packaged with one or more flavors that is separate from the food. The flavors are chosen by the consumer and added to the food. In this group consumers just add separate flavors directly to the food or beverage that they are consuming.

Therefore it can be seen that the prior art consists of adding a flavor or aroma directly to food by either mixing it directly into that food or incorporating it into the packaging or utensils used in consumption of the food.

Document WO2013/144836 discloses a device and method for priming a person (Abstract). The device includes a detector for detecting when a person expresses or should express a predetermined behavior, a stimulus unit for providing a sensory stimulus when it has been detected that the person expressed a predetermined behavior, and a control unit for controlling the stimulus unit. The purpose of document WO2013/144836 is to provide a device and method for priming a person to support a healthy lifestyle management, and to support the willingness of the person to engage in a desired behavior (page 1, line 28 to page 2, line 3).

Document US2013/0302477 discloses consumable items provided with scented packaging and using scent to reduce ingredients in consumable items (Title). A beverage and packaging includes a container configured to hold the beverage (paragraph [0006]). The beverage includes and ingredient having a characteristic taste, and a component of packaging including aromatic material. The purpose of document US2013/0302477 is to provide improved packaging systems that enable the efficient provision of gas and other phases of a consumable item (paragraph [0005]).

Document WO2007/017742 discloses a device for projecting and directing a scent (Abstract). A device includes a drive mechanism, a housing cap, an air projector removably connected to a shaft of the drive mechanism, and a scent-releasing substance. The stated purpose of document WO2007/017742 is to use scents to improve the smell of a user's environment, and more particularly, to provide a device capable of projecting and directing a scent within an environment that is substantially perceived only be the user (paragraph [0002]).

Document US 7,376,344 discloses a scent delivery device that employs a housing and a scent generator which selectively releases scent (Abstract). The housing is mounted on a counter and allows a user to selectively sample a variety of scents. The scent travels to the user's nose by diffusion. The purpose of document US 7,376,344 is to provide "a miniaturized stationary scent delivery device that can be easy allowing a user to sample a scent by diffusion" (col. 1, lines 36-38).

Document WO2005/099663 discloses devices and methods for creating olfactory perception for a person in a virtual reality environment by introducing scents in synchronism with visual and/or acoustic representations (page 2, line 30 to page 3, line 12). In one embodiment, a scent cartridge includes a scent reservoir having a basin, a lid and a scented material in the basin; a housing defining an enclosure; a valve connected to the lid; and a fan. The stated purpose of document WO2005/099663 is to provide a scent delivery device that can be used to enhance emotional experiences in virtual environments (page 2, lines 2-5).

Document US 2009/0216070 discloses apparatus and methods for contemporaneously displaying video images, delivering audio signals and emitting a scent to an individual (Abstract). A visual sensory portion includes a video screen. An auditory sensory portion includes a speaker. An olfactory sensory portion includes a renewable scent element impregnated with an aroma for emitting a scent to the user. The purpose of document US 2009/0216070 is to provide an apparatus and method for providing video images, audio signals, and emitting a scent to an individual in connection with relaxation therapy (paragraph [0005]).

### Summary of the Invention

An object of the present invention is to provide a scent to a person where the scent is provided by a scent delivery device which is independent and separate from the food, the packaging for the food or a utensil for the food so as to affect enjoyment of the food while the person consumes the food. Specifically, the effect is an increased enjoyment of food, or a decreased enjoyment of food.

The term food also means foodstuff, and includes any substance intended to be or reasonably expected to be ingested by humans. Food includes drinks, beverages, chewing gum, candy and other edibles. It does not include animal feed, live animals, plants prior to harvest, cosmetics, tobacco products nor narcotics.

Increased enjoyment of food in accordance with the present Invention is accomplished by providing a food to the consumer and delivering a scent to the consumer with the scent delivery device. The scent enhances, emulates, modulates or alters the perceived flavor of the food. The perceived flavor can be modified by providing a flavor different than the food. For example, the consumer is provided chocolate ice cream and a peppermint scent is delivered. This simulates the eating of peppermint chocolate ice cream. The perceived flavor of the food can be emulated by providing a low quality meat food and delivering a high quality meat scent. This simulates the eating of high quality meat. The scent can be enhanced by increasing the amount of scent associated with the food to increase the desire to eat the food and thus be used to treat anorexic individuals.

Decreased enjoyment of food in accordance with the present Invention is accomplished by providing a food to the consumer and delivering a scent that has no relationship or association with the food. This diminishes the flavor of the food which, in turn, decreases the enjoyment and consumption of the food. This can be used to treat obese individuals.

A scent delivery device means any dedicated device intended for providing one or more scents to a person simultaneously or in sequence in a way that is controllable by the person using that device. The scent delivery device is a dedicated device that can deliver scent without the collaboration of any other processes. More specifically, the device delivers scent without the scent being added to the food or added to the utensils used to consume the food or the packaging used to store the food.

Increased enjoyment of food can be further enhanced by providing a tastant of the food to the user. A tastant is defined in Stedman's Medical Dictionary as: "any chemical that stimulates the sensory cells in a taste bud. Taste buds contain the receptors for taste. They are located around the small structures on the upper surface of the tongue, soft palate, upper esophagus and epiglottis, which are called papillae. These structures are involved in detecting the five (known) elements of taste perception: salty, sour, bitter, sweet, and umami." It is the gustatory (appreciated by the tongue) equivalent of a scent molecule which stimulates the olfactory bulb. Taste is the result of the combination of the effect of tastants and scent molecules.

In general all taste is the result of two components. One component stimulates the taste buds on the tongue. It turns out that there are only 5 tastes that the tongue can appreciate: sweet, sour, salty, bitter and umami (a taste identified by the Japanese). The rest of taste is actually mediated by the olfactory receptors. When food is chewed aliphatic and aromatic compounds are released at the posterior pharynx. These volatile compounds diffuse up the nasopharynx and stimulate receptors in the olfactory bulb.

Thus, all foods can be deconstructed into the taste bud components and the olfactory components. Additionally, the magnitude of taste bud components of sweet, sour, salty, bitter and umami can be determined. Then the component in the food which rise up to stimulate the olfactory bulb can be determined. It is the olfactory component that is the focus of the scent delivery device of the present invention.

The present invention also allows food to be presented to the consumer in an entirely new way. That is foods can be packaged so as to provide texture and the requisite taste bud component of sweet, sour, salty, bitter and umami. The rest of the food delivery can occur with a separate scent delivery device in accordance with the present Invention. The same populations of aromatic, aliphatic and other volatile components which diffuse up the posterior naxopharynx can now be delivered with an external scent delivery device. This process broadly widens the ability and flexibility to provide flavor and taste by the food producer.

Specifically, food substitutes can be employed using the present Invention wherein the aroma is provided to effect the enjoyment of the food substitute. Food substitutes are typically food that has been depleted of unwanted elements such as fat, sugar and salt. Ingesting them by using the scent delivery device of the present Invention with or without possible tastants to emulate the taste of the original food allows for an inexpensive and healthful way of ingesting food while avoiding unwanted elements such as fat, sugar, and salt, etc.

Preferably food substitutes are other foodstuffs. These foodstuffs will have similar texture and mechanical properties of the food it is substituting for but have much fewer calories, fat, sugar and any other constituents which are to be avoided for health or other dietary reasons. This does not preclude the use of non food substitutes which would also have food like textures and mechanical properties such as a synthetic cellulosic noodle which would have the firmness and flexibility like pasta but would be completely indigestible. For example "Olean" a fat substitute made by Proctor and Gamble has the mouth feel of oil but it is completely nondigestble.
Four Examples of food substitutes are:
1) Seitan: Made from Wheat gluten. Recipe widely available . Used as meat substitute. Contains almost no fat and no cholesterol.
2) Soft Tofu can be used as egg substitute, as a sauce base.
3) Very low calorie bread made with a high percentage of flax fiber which can make bread like texture but is very low in calories. An example is Earth Grains Bread, 35 Reduced Caloric.
   Ingredients:
   Enriched Bleached Flour [Wheat Flour, Malted Barley Flour, Niacin, Iron, Thiamin Mononitrate (Vitamin B1), Riboflavin (Vitamin B2), Folic Acid], Water, Wheat Gluten, Cellulose, High Fructose Corn Syrup, Yeast.
   Contains 2% Or Less of Each of The Following: Whole Wheat Flour, Salt, Wheat Bran, Yeast Nutrients (Monocalcium Phosphate, Calcium Sulfate, Ammonium Sulfate), Dough Conditioners (May Contain One Or More of The Following: Mono- and Diglycerides, Ethoxylated Mono- and Diglycerides, Sodium Stearoyl Lactylate, Ascorbic Acid, Azodicarbonamide, Enzymes), Corn Starch, Calcium Propionate (Preservative), Distilled Vinegar, Caramel Color, Soy Lecithin, Soy Flour. Contains Wheat and Soy.
4) Shirataki, very low carbohydrate, low calorie, thin, translucent, gelatinous traditional Japanese noodles made from devil's tongue yam (elephant yam or the konjac yam). The word "shirataki" means "white waterfall", describing the appearance of these noodles.

Largely composed of water and glucomannan, a water-soluble dietary fiber, they have little flavor of their own.

The invention relates to a method for affecting enjoyment of food according to claim 1.

The invention can be defined by one or more of the following items.
1. A method for affecting enjoyment of food, comprising:
   providing a scent delivery device to a user; wherein the scent delivery device is a head set that comprises a fan having adjustable speed, a scent container holding a scent component, and a nozzle attached at one end to the fan and at the other end to the scent container, and;
   providing a food to the user; and
   delivering the scent to the user by creating a current of air directed towards the nose or face of the user while the user cosumes the food in order to affect the enjoyment of the food.
2. The method of item 1, wherein
   the effect is an increased enjoyment or a decreased enjoyment of the food.
3. The method of item 2, wherein
   the effect is increased enjoyment of the food.
4. The method of item 3, wherein
   the scent is associated with the food to cause increased consumption of the food by the user.
5. The method of item 3, wherein
   the scent is different than the food thereby changing the perception of the user of the food being consumed by the user.
6. The method of item 2, wherein
   the effect is decreased enjoyment of the food.
7. The method of item 1, wherein
   the effect is increased enjoyment or decreased enjoyment; and the method further comprises:
   providing a food to the user;
   delivering a scent from the scent delivery device to the user before providing the food to the user; and
   continuing to deliver scent from the scent delivery device to the user after the food is provided to the user and during the consumption of the food by the user.
8. The method of item 1, wherein
   two or more scents are delivered to the user by the scent delivery device.
9. The method of item 1, wherein
   the two or more scents are delivered either simultaneously or sequentially.
10. The method of item 3, further comprising:
   providing a tastant of the food to the user for adding to the food during consumption of the food by the user.
11. The method of item 2, further comprising:
   delivering a tastant of the food to the user simultaneously with delivering a scent from the delivery device to the user.
12. The method of item 11, wherein
   the tastant is delivered to the mouth of the user by a taste strip or microcapsule.
13. The method of item 1, wherein the food is candy.
14. The method of item 1, wherein the food is a beverage.
15. The method of item 13, wherein the candy is chocolate.

### Brief Description of the Drawings

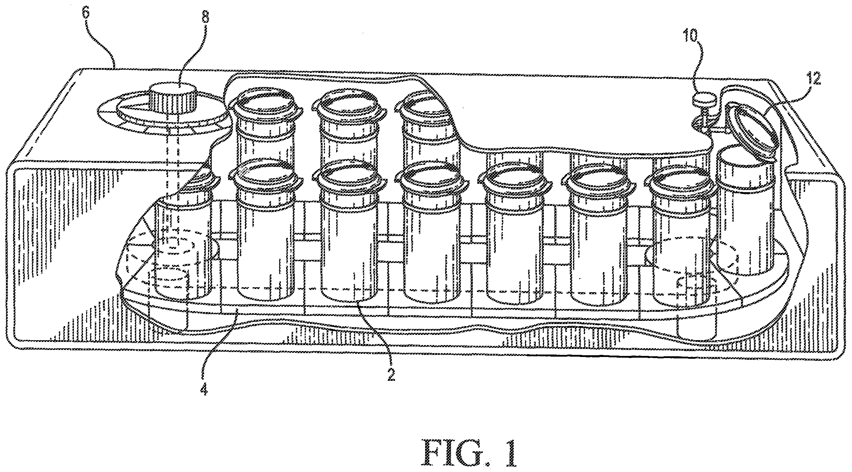

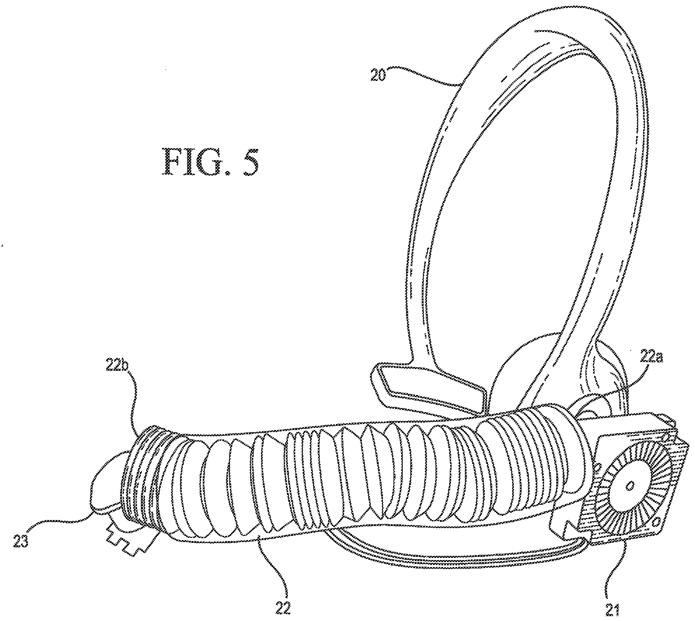

Figs. 1-5 illustrate scent delivery devices. Figure 5 only illustrates the scent delivery device for use in the present Invention.

### Detailed Description of the Invention

Scent delivery devices have been disclosed. For example, air freshening devices are sold commercially which provide scented air. These devices often have heaters and fans to help deliver the scent. Examples of such scent delivery devices are sold under the Trademarks GLADE by S. W. Johnson Company; FEBREEZE Set and Refresh by The Proctor and Gamble Company; and AIR WICK by Reckitt and Colman.

Scent delivery devices are taught in U.S. Patent Nos. 6,803,987; 7,203,417; 7,036,502; and 7,376,344. These devices provide scent to a person and have the ability to provide multiple scents both simultaneously and sequentially.

FIG. 1 illustrates one embodiment of a delivery means. Scent containers 2 are mounted on rotatable endless belt 4 within housing 6. Rotatable dial 8 extends through the surface of housing 6 in order to provide a mechanism to manually rotate endless belt 4 and scent containers 2. Push button 10 extends through the surface of housing 6 in order to provide a mechanism to manually open flap 12 of a scent containers 2 that is located beneath push button 10. When push button 10 is depressed, flap 12 of the corresponding scent container 2 is lifted to release the scent trapped within scent container 2. When push button 10 is released, flap 12 of scent container 2 is lowered to trap the scent within scent container 2. It will be appreciated that a plurality of push buttons 10 can be positioned throughout housing 6 to allow for flaps 12 of multiple scent containers 2 to be lifted simultaneously.

It is important to tightly close flap 12 in order to facilitate the tight closing, a spring and/or piston can be employed. If flap 12 is not tightly closed, scent can escape.

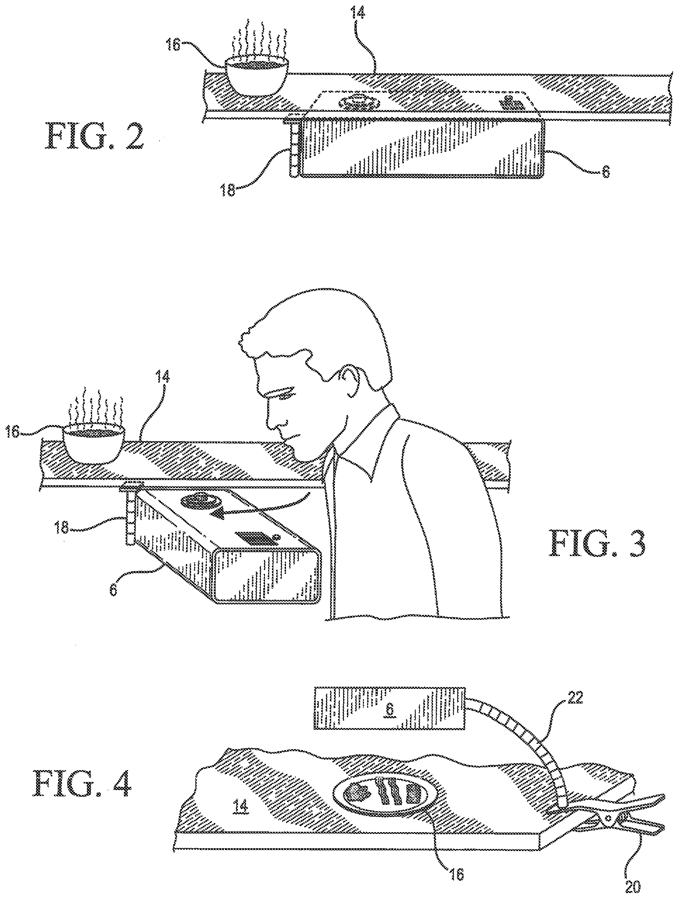

FIGS. 2 and 3 illustrate an attachment of the delivery means of FIG. 1 to a position beneath dining table 14 having food 16. Rotating mechanism 18 is attached to housing 6 to provide a rotation of the entire device from a hidden position beneath table 14 to an extended position from table 14. Rotating mechanism 18 provides for the conservation of space when the device is not in use. Rotating mechanism 18 can also be attached to table 14 at a position above table 14 to provide a rotation of the entire device above table 14.

It will be appreciated that dial 8 can be replaced with an electrical motor, associated sensors and a microcontroller to facilitate movement of the belt. Push button 10 and its associated mechanism can likewise be driven by an electrical motor, microcontroller and sensors.

As shown in Fig. 4, the scent delivery device of FIG. 1 includes housing 6 releasably affixed to table 14 by means of clamp 20 and flexible gooseneck 22. Flexible gooseneck 22 allows a user to move housing 6 while clamp 20 remains affixed to table 14. Clamp 20 is a spring clamp which allows a user to remove the device and affix the device to other stationary objects.

In the embodiment that employ a moving belt or moving disc to open and close the scent containers, sensors are employed to provide feedback as to the location of the scent containers to allow the processor to control the placement and to know the location of the scent container.

As shown in FIG. 5, the scent delivery device of the present invention is a head set 20 including a blower fan 21 and a nozzle 22. One end 22a of the nozzle 22 is attached to the blower fan 21. The other end 22b of the nozzle 22 is attached to a vial 23 containing an aroma component. The nozzle 22 can be a flexible boom tube which allows for adjustment of position of the end 22b of the nozzle 22 and the vial 23. The blower fan 21 can be a mini blower fan having the size of 30 mm x 30 mm x 10 mm. For example, a fan "Delta BFB0312HA" can be used with maximum flow rate of 1.45 cfm in combination with 12 volt DC source and drawing 0.09 amps power. The vial 23 can be a 15 ml vial from LA containers. In this embodiment, the fan is turned on when a food is ingested. The fan speed can be adjusted with a potentiometer (for example, 10K ohms) to vary the flow rate from about 0 cfm to 1.45 cfm. The intensity of the scent may be inversely related to the flow rate. The flow rate can be adjusted by a consumer for the desired effect.

The head set can include a tastant supplier to supply a tastant separately from the scent. For example, the tastant supplier includes a tastant container and a nozzle for delivering a solution of the tastant. The solution of the tastant can be sprayed from the nozzle to a consumer's mouth or directly supplied to a consumer's mouth. The nozzle for tastant is separate from the nozzle for scent.

A fan and/or heater can be used in association with the scent delivery device to create a current of air directed towards the nose or face of the user and thereby assist in the diffusion of an aroma component to the nose. Suitable fans include blowers, centrifugal or axle fans. Such fans are conventional fans which are small in size. One such fan measures 1 1/2 inches x 1 1/2 inches by 1 inch (4 x 4 x 2.5 cm) and moves 7 cubic feet (0.1981 m³) of air per minute. Heater can be selected from conventional heaters which are small in size. Heater can heat the air flow from the fan and/or a container of an aroma component to deliver the scent.

Further disclosed are scent delivery devices that can include a spray or an atomizer as the scent supplier to deliver an aroma component. For example, an aroma component which is susceptible to heat can be delivered with the spray without heating.

The time period of air flow from the scent delivery device can be controlled. In one embodiment, a consumer can turn on a blower to deliver the scent at the beginning of meal and turn off the blower at the end of meal. In another embodiment, the device can include a motion sensor which can detect a consumer's movement of chewing or drinking, and control the air flow so as to deliver the scent only when a food or beverage is in the consumer's mouth.

The air flow rate can be controlled. In one embodiment, a consumer can adjust the air flow with a potentiometer of the scent delivery device. In another embodiment, the device can include a motion sensor which can detect at least two types of consumer's movements, one of which increases the flow rate and another of which decreases. For example, when using the scent delivery device which is a head set, a motion sensor can detect a nod to increase the flow rate, and inclining the consumer's head decreases the flow rate.

The processor and the battery for the device of the present Invention can be made internal or external to the device. For example, they are housed and connected by conventional wires to the device.

In one embodiment, scents that are suitable depend on the food and the desired effect. For example, a flavorist or flavor chemist can prepare or choose a scent component which is suitable to each food. The flavorist is a person skilled in the field of scent for foods. In another embodiment, a consumer can choose his or her favorite or desirable scent when consuming a food.

The scent employed to increase enjoyment is a scent that reinforces the flavor of the food. For example, when consuming vanilla ice cream, the delivered scent can be vanilla. The consumer can enjoy the ice cream more with the reinforced flavor of vanilla.

To increase enjoyment of foods, the scent can also change the flavor of food. For example, peppermint scent is provided to the consumer during the consumption of chocolate ice cream to provide the consumer with the sensation/perception of eating mint chocolate ice cream. The consumer can firstly enjoy the chocolate ice cream without the peppermint scent and then use the peppermint scent to change the flavor of the chocolate ice cream. By doing so, the consumer can enjoy two types of flavors by just adding the peppermint scent.

The effect can be an increased enjoyment of food where the scent provides flavor to the food. For example, a peppermint scent is provided when eating an ice cream with no flavor.

In all cases of increased enjoyment, the scent delivered is a scent which is associated with or conventionally used for or with food. Thus, the scent is one normally associated with food. For example, the scent of leather, although appealing, is not a scent which is delivered in accordance with the present Invention for increased enjoyment of food.

However, a new flavor that was not found in a preexisting food or beverage can be used. Here, the scent molecules delivered during consumption of the food have never been associated with that food before. This therefore provides for the creation of new flavors that are novel and not known to those experienced in the culinary arts or flavor production. For example, the smell of red pepper can be provided with plain ice cream to provide a new ice cream flavor.

Any conventional scent or aroma component can be used and such are conventionally available. Natural aroma component, artificial aroma component, or their combination can be used.

The natural aroma component can be isolated or extracted from natural source such as animals and plants including fruits, vegetables, oil, flowers, beans, herbs, spices, dairies, and microorganism. The isolation and extraction of the natural aroma component can be performed by any conventional method such as distillation, liquid extraction, and gas extraction.

The artificial aroma component can be a synthetic compound which is chemically identical to one found in nature or a compound which is not found in nature. The artificial aroma component can be prepared by any conventional synthetic or enzymatic reaction. Examples of the aroma compounds include, but not limited to, carbonyl compounds, pyranones, furanones, thiols, thioethers, disulfides, trisulfides, pyrroles, pyridines, pyrazines, amines, phenols, alcohols, hydrocarbons, esters, lactones, and terpenes.

The aroma compounds described in H.-D. Belitz, et al, Food Chemistry, Springer 2009 and W. Grosch, Chem. Sences 26; 533-545, 2001 can be used in this Invention. Also, the method of preparing aroma compounds in these articles can be used.

For decreased enjoyment of food, a scent is employed which is not normally associated with food. The scent need not be offensive to the user for decreased enjoyment of the food. For example, the smell of leather can be used. Although the smell of leather is not offensive, it decreases enjoyment of food. This decreased enjoyment can result in a decreased consumption and thus be used to treat obesity.

The aroma compounds can be used in this Invention as a solution of, for example, water, alcohol, oil, or other acceptable organic solvents. The solution can be an essential oil or absolute oil. Also, for example, ethanol, triethyl citrate, dipropylene glycol, and benzyl benzoate can be used as a solvent.

The solution of the aroma compounds can be used at any suitable concentration. However, the concentration is at least odor threshold which is the lowest concentration of an aroma compound that is just enough for the recognition of the scent of the compound. The threshold concentration depends on each compound's volatility, which is effected by both temperature and medium. Also, each consumer's sensitivity can affect the threshold concentration.

The aroma component is provided independent of the comestible. Suitably, in a separate package from the comestible or sold totally independent of the comestible.

The amount of scent delivered depends on the volume of space that needs to be filled in order for a consumer to perceive the scent. If the scent would be delivered through a nasal tube, a low volume of scent would be needed because the scent is delivered directly to the nose. If the scent would be delivered by a device with scent outlet spaced two feet from the consumer, then a larger volume of scent would be needed for the consumer to perceive the scent.

The amount of scent is also dependent upon the consumer. Different people have different sensitivities to scent. Thus, the amount of peppermint scent delivered to one person to perceive mint chocolate ice cream when eating chocolate ice cream can be different than another person's need for the amount of peppermint scent to obtain the same perception.

Thus, the device for delivering the scent is adjustable or controllable for the amount of scent to be delivered. Since the scent is delivered through a flow of air, the volume of scented air outputted by the device is controlled to control the amount of scented air delivered to the consumer. Also, the intensity of scent may be inversely related to the air flow rate. The flow rate can be adjusted by a consumer for the desired effect.

Also, the amount of scent from the aroma component can be adjusted by controlling the temperature of the aroma component. The higher the temperature is, the more aroma component volatilizes. In order to control the temperature of the aroma component, the scent delivery device can include a heater for the aroma component.

Most aroma components have a maximum amount that can be suspended in air and this saturation point is dependent upon the moisture content and temperatures of the air. Generally, moist, hot air (75% humidity, 75°F) holds more scent than dry, cold air (10% humidity, 32°F). The device can add both heat and moisture to the air to increase the concentration of scent in the air flow. Thus, the device can be adjustable not only for the volume of air outputted, but also for the temperature and humidity of the air.

The humidity and temperature of the air outputted by the device is accomplished in a conventional manner, such as adding a heater to the device and a source of water, similar to a humidifier.

The tastant is a component for a consumer to perceive sweet, sour, salty, bitter, umami, or a combination of two or more of these tastes. The tastant for sweet can be any conventional sweetener such as sugar, sugar alcohol, stevia, and artificial sweetener including aspartame, acesulfame potassium, sucralose, and saccharin. The tastant for sour can be any conventional edible acid such as acetic acid, citric acid, succinic acid, lactic acid, malic acid, tartaric acid, gluconic acid, and phosphoric acid. The tastant for salty can be sodium chloride. The tastant for bitter can be selected from conventional food additives for bitter such as caffeine, spice, and naringin. The tastant for umami can be any conventional
food additive for umami such as glutamate, and guanylate, inosinate.

The amount and type of tastant will depend on the level of taste and the volume of the food being consumed. The amount and type of tastant can be determined by each consumer. Alternatively, for example, an electronic tongue can be used as described in Latha et al., J. Adv. Pharm... Technol. Res. 2012 Jan-Mar; 3(1):3-8. The addition is preferably controlled by the user by providing a separate package of the tastant with the food.

The deconstruction of a food into a taste bud component and an olfactory component is done in a conventional manner using conventional equipment. This separated olfactory component can be used as the scent to increase enjoyment of food.

Below are some suggested scents and foods which can be used together to either increase enjoyment or decrease enjoyment of food. This list is not exhaustive but is exemplary.

**INCREASED ENJOYMENT**

| **Scent** | **Food** |
|---|---|
| Mint | Ice Cream |
| Mint | Chocolate |
| Strawberry | Vanilla Ice Cream |
| Lemon | Tea |
| Vanilla | Cola, soda |

**DECREASED ENJOYMENT**

| | |
|---|---|
| Leather | Steak |
| Lavender | Milk |
| Lilly | Ice cream |

These and other aspects of the present invention may be more fully understood by reference to one or more of the following examples.

### Example I

This is an example of increased enjoyment of food in accordance with the present invention.

Consumers were given plain chocolate to taste. They then were given the chocolate to consume again but this time the scent of peppermint was delivered to their noses while they ate the chocolate. They experienced this process as if they were consuming mint chocolate.

### Example II

This is another example of increased enjoyment of food in accordance with the present invention.

Consumers were given plain salted popcorn to consume. It had a light spray of a neutral oil (e.g. canola) to approximate the "feel" of butter in their mouth, but there was no butter taste. They then consumed the same popcorn a second time, however, the second time the scent of butter was delivered to their nose while they were eating the popcorn. They experienced this process as if they were actually eating buttered popcorn.

In this example, the tastant added will also enhance the enjoyment of the popcorn. The tastant can be the sweet, or sour, or salty component of the butter or a combination of two or more of these tastants.

### Example III

This is yet another example of increased enjoyment of food in accordance with the present invention.

Salad is served with a "generic dressing". The generic dressing had a little bit of oil, some salt and a little vinegar.

The scent of garlic is delivered while salad and generic dressing is being eaten. The dressing tasted like Caesar dressing.

Next day the consumer used the same salad and generic dressing and with a different scent, ranch. The consumer perceives they are eating salad with ranch dressing.

This same procedure can be followed such that each day the generic dressing is used but a different scent is employed to make the consumer perceive they are eating a different salad dressing. For example, the scents can be honey mustard one day and raspberry another day.

### Example IV

This is still a further example of the increased enjoyment of food in accordance with the present invention.

A food's flavor can be easily altered by changing its smell while keeping its taste similar. This is exemplified in artificially flavored jellies, soft drinks and candies, which, while made of bases with a similar taste, have dramatically different flavors due to the use of different scents or fragrances. Dazzling results can be achieved by providing a consumer with a candies which all have the same base (taste components). When the candy is placed in the mouth a whole variety of different flavors is achieved by delivering different scent mixtures which correspond to the aromatic compounds in those different flavors. In other words one can quickly change from grape to cherry to banana by just using a scent delivery device to deliver different combinations of scents.

## Claims

1. A method for affecting enjoyment of food, comprising:
providing a scent delivery device to a user; wherein the scent delivery device is a head set that comprises a fan having adjustable speed, a scent container holding a scent component, and a nozzle attached at one end to the fan and at the other end to the scent container, and;
providing a food to the user; and
delivering the scent to the user by creating a current of air directed towards the nose or face of the user while the user consumes the food in order to affect the enjoyment of the food.

2. The method of Claim 1, wherein
the effect is selected from an increased enjoyment and a decreased enjoyment of the food.

3. The method of Claim 2, wherein
the effect is increased enjoyment of the food.

4. The method of Claim 3, wherein
the scent is associated with the food to cause increased consumption of the food by the user.

5. The method of Claim 3, wherein
the scent is different than the food thereby changing the perception of the user of the food being consumed by the user.

6. The method of Claim 2, wherein
the effect is decreased enjoyment of the food.

7. The method of Claim 1, wherein
the effect is increased enjoyment or decreased enjoyment; and the method further comprises:
delivering a scent from the scent delivery device to the user before providing the food to the user; and
continuing to deliver scent from the scent delivery device to the user after the food is provided to the user and during the consumption of the food by the user.

8. The method of Claim 1, wherein
two or more scents are delivered to the user by the scent delivery device.

9. The method of Claim 1, wherein
the two or more scents are delivered either simultaneously or sequentially.

10. The method of Claim 3, further comprising:
providing a tastant of the food to the user for adding to the food during consumption of the food by the user.

11. The method of Claim 2, further comprising:
delivering a tastant of the food to the user simultaneously with delivering a scent from the delivery device to the user.

12. The method of Claim 11, wherein
the tastant is delivered to the mouth of the user by a taste strip or microcapsule.

13. The method of claim 1, wherein the food is candy.

14. The method of claim 1, wherein the food is a beverage.

15. The method of claim 13, wherein the candy is chocolate.

## Patentansprüche

1. Verfahren zur Beeinflussung des Genusses von Lebensmitteln, umfassend:
Bereitstellen einer Duftabgabevorrichtung an einen Verbraucher; wobei die Duftabgabevorrichtung ein Headset ist, dass einen Ventilator mit einstellbarer Geschwindigkeit, einen Duftbehälter, der eine Duftkomponente enthält, und eine Düse, die an einem Ende am Ventilator und am anderen Ende am Duftbehälter befestigt ist, umfasst, und;
Bereitstellen eines Lebensmittels an den Verbraucher; und
Abgabe des Duftes an den Verbraucher durch Erzeugen eines auf die Nase oder das Gesicht des Verbrauchers gerichteten Luftstroms, während der Verbraucher das Lebensmittel verzehrt, um den Genuss des Lebensmittels zu beeinflussen.

2. Verfahren nach Anspruch 1, wobei die Wirkung aus einem erhöhten Genuss und einem verminderten Genuss der Lebensmittel ausgewählt ist.

3. Verfahren nach Anspruch 2, wobei die Wirkung ein erhöhter Genuss des Lebensmittels ist.

4. Verfahren nach Anspruch 3, wobei der Duft dem Lebensmittel zugehörig ist, um einen erhöhten Verzehr des Lebensmittels durch den Verbraucher zu bewirken.

5. Verfahren nach Anspruch 3, wobei sich der Duft von dem Lebensmittel unterscheidet und dadurch die Wahrnehmung des vom Verbraucher verzehrten Lebensmittels beim Verbraucher verändert wird.

6. Verfahren nach Anspruch 2, wobei die Wirkung ein verminderter Genuss des Lebensmittels ist.

7. Verfahren nach Anspruch 1, wobei die Wirkung ein erhöhter oder verminderter Genuss des Lebensmittels ist; und das Verfahren ferner umfasst:
Abgabe eines Duftes aus der Duftabgabevorrichtung an den Verbraucher vor der Bereitstellung des Lebensmittels an den Verbraucher; und
Weitere Abgabe eines Duftes aus der Duftabgabevorrichtung an den Verbraucher nach der Bereitstellung des Lebensmittels an den Verbraucher und während des Verzehrs des Lebensmittels durch den Verbraucher.

8. Verfahren nach Anspruch 1, wobei durch die Duftabgabevorrichtung zwei oder mehr Düfte an den Verbraucher abgegeben werden.

9. Verfahren nach Anspruch 1, wobei die zwei oder mehr Düfte entweder gleichzeitig oder nacheinander abgegeben werden.

10. Verfahren nach Anspruch 3, ferner umfassend:
Bereitstellen eines Geschmacks des Lebensmittels an den Verbraucher zur Bereicherung des Lebensmittels, während der Verbraucher das Lebensmittel verzehrt.

11. Verfahren nach Anspruch 2, ferner umfassend:
Abgabe eines Geschmacks des Lebensmittels an den Verbraucher gleichzeitig mit der Abgabe eines Duftes aus der Abgabevorrichtung an den Verbraucher.

12. Verfahren nach Anspruch 11, wobei der Geschmack durch einen Geschmacksträger in Streifenform oder eine Mikrokapsel in den Mund des Benutzers abgegeben wird.

13. Verfahren nach Anspruch 1, wobei das Lebensmittel ein Süßwarenprodukt ist.

14. Verfahren nach Anspruch 1, wobei das Lebensmittel ein Getränk ist.

15. Verfahren nach Anspruch 13, wobei das Süßwarenprodukt Schokolade ist.

## Revendications

1. Méthode pour influer sur le plaisir alimentaire, comprenant :
fournir un dispositif de délivrance d'odeur à un utilisateur ; dans lequel le dispositif de délivrance d'odeur est un ensemble de tête qui comprend un ventilateur ayant une vitesse réglable, un récipient d'odeur contenant un composant d'odeur, et une buse fixée dans une extrémité au ventilateur et dans l'autre extrémité au récipient d'odeur, et ;
fournir un aliment à l'utilisateur ; et
délivrer l'odeur à l'utilisateur en créant un courant d'air dirigé vers le nez ou le visage de l'utilisateur pendant que l'utilisateur consomme l'aliment afin d'influer sur le plaisir alimentaire.

2. Méthode selon la revendication 1, dans laquelle
l'effet est choisi parmi un plaisir alimentaire accru et un plaisir alimentaire diminué.

3. Méthode selon la revendication 2, dans laquelle
l'effet est un plaisir alimentaire accru.

4. Méthode selon la revendication 3, dans laquelle
l'odeur est associée à l'aliment pour provoquer une augmentation de la consommation de l'aliment par l'utilisateur.

5. Méthode selon la revendication 3, dans laquelle
l'odeur est différente de celui de l'aliment, modifiant ainsi la perception de l'utilisateur de l'aliment étant consommé par celui-ci.

6. Méthode selon la revendication 2, dans laquelle
l'effet est une diminution du plaisir alimentaire.

7. Méthode selon la revendication 1, dans laquelle
l'effet est un plaisir accru ou un plaisir diminué ; et la méthode comprend en outre :
délivrer une odeur à l'utilisateur à partir du dispositif de délivrance d'odeur avant de fournir l'aliment à l'utilisateur ; et
continuer à délivrer l'odeur à partir du dispositif de délivrance d'odeur à l'utilisateur après que l'aliment a été fourni à l'utilisateur et pendant la consommation de l'aliment par l'utilisateur.

8. Méthode selon la revendication 1, dans laquelle
deux ou plusieurs odeurs sont délivrés à l'utilisateur par le dispositif de délivrance d'odeur.

9. Méthode selon la revendication 1, dans laquelle
les deux ou plusieurs odeurs sont délivrés soit simultanément soit successivement.

10. Méthode selon la revendication 3, comprenant en outre :
fournir un agent aromatisant de l'aliment à l'utilisateur pour ajouter à l'aliment pendant la consommation de l'aliment par l'utilisateur.

11. Méthode selon la revendication 2, comprenant en outre :
délivrer un agent aromatisant de l'aliment à l'utilisateur simultanément avec la délivrance d'une odeur à partir du dispositif de délivrance à l'utilisateur.

12. Méthode selon la revendication 11, dans laquelle
l'agent aromatisant est délivré à la bouche de l'utilisateur par une bande gustative ou une microcapsule.

13. Méthode selon la revendication 1, dans laquelle l'aliment est un bonbon.

14. Méthode selon la revendication 1, dans laquelle l'aliment est une boisson.

15. Méthode selon la revendication 13, dans laquelle le bonbon est du chocolat.
